# EUROPEAN PATENT SPECIFICATION

(11) **EP 2 682 153 B1**
(45) Date of publication and mention of the grant of the patent: **06.09.2017**
(21) Application number: 13175144.8
(22) Date of filing: 04.07.2013
(51) Int. Cl.: A61M 25/10

(54) **Device for treatment of a blood vessel**
Vorrichtung zur Behandlung eines Blutgefäßes
Dispositif de traitement d'un vaisseau sanguin

(30) Priority: 05.07.2012 IT MI20121183
(43) Date of publication of application: 08.01.2014
(73) Proprietor: Merit Medical Systems, Inc., South Jordan, UT 84095 (US)
(72) Inventor: Cremascoli, Paolo, 20122 Milan (IT); Silva, Pedro Orrego, 20125 Milan (IT); Pini, Patrizia, 22060 Novedrate (CO) (IT)
(74) Representative: Baroni, Matteo

(56) References cited:
- WO-A1-02/089899
- US-A- 5 599 307
- US-A- 5 833 658
- US-A1- 2008 221 552

## Description

### FIELD OF THE INVENTION

The present invention refers to a device for treating a blood vessel.

### STATE OF THE ART

Coronary artery disease is the most common form of heart disease in Europe and the United States, and currently is the leading cause of death worldwide, affecting equally both men and women. Coronary artery is a disease of the heart caused by a buildup of fatty deposits of waxy (cholesterol, calcium, etc.) on the inner walls of arteries.

The bundling of such deposits determines the formation of atherosclerotic plaques that obstruct the coronary arteries causing the so-called hardening of the arteries or atherosclerosis, with consequent narrowing of the lumen of the arteries themselves, thus reducing the supply of blood and oxygen to the heart muscle. The narrowing and obstructing of the coronary arteries prevents the heart from receiving amounts of oxygen and nutrients sufficient for its regular operation, and in the most severe cases can lead to a progressive death of cardiac muscle, i.e. the myocardial infarct.

When the narrowing and the obstruction of the coronary arteries (so-called stenosis) occurs, it can be treated by angioplasty, which is an intervention aimed at restoring the direct circulation to the heart. Coronary Angioplasty (PTCA) is usually the fastest and most modem treatment to reopen a blocked artery and is, according to the guidelines of the European Society of Cardiology (ESC) published in November 2008, the technique of excellence in the event of heart attack infarction.

Angioplasty is an interventional procedure which is less invasive than other surgical procedures, since it does not involve opening the chest and using general anesthesia; the cardiologist that operates the procedure makes a small skin incision suitable for the insertion of a small catheter in an artery in the groin (femoral artery) or in the region of the wrist (radial artery). This procedure is performed in cathlab, using an angiograph, and the analysis of the coronaries is performed by means of a contrast medium. The occluded coronary artery is opened, in the PTCA (Percutaneous Transluminal Coronary Angioplasty), by inserting the catheter, which is made of a small hollow, long and flexible tube, which supports a balloon at one of its end, in the femoral or radial artery. The balloon catheter is inserted by Seldinger technique to reach the coronary artery where the narrowing due to the atherosclerotic plaques is located, and when it arrives at the stenosis it is expanded causing plaques compression and stretching the artery wall. When the plaque has been sufficiently compressed and the artery has been re-opened, the balloon is deflated and removed.

The angioplasty balloon is not "compliant" and is inflated to about 6 atmospheres.

The angioplasty has a higher safety and efficacy if followed by stenting, i.e. the application of a coronary stent, a small tube having a metal net structure, adapt to provide a support to the dilated artery, to favor the patency and to avoid a new shrinkage of the same artery.

The stent is positioned on a catheter having a balloon at one of its ends and advanced along the guidewire to the selected artery. When it reaches the targeted location, the inflation of the balloon allows stent expansion which causes a compression of atherosclerotic plaques against the artery wall. At the end of the procedure and performed the complete inflation of the stent, the catheters and the guidewire are removed, whereas the stent stays implanted within the artery in order to prevent re-obstruction.

Currently, the majority of coronary angioplasty procedures are executed along with the placement of coronary stents and often also with the procedures in "direct stenting", that is the expansion of the stent without a previous dilatation of the balloon.

Coronary angioplasty and the application of stents are certainly effective interventions and they are able to successfully treat coronary heart disease but they may present complications such as the restenosis, that is the appearance of scar tissue around the metal net structure of the stent during a process called neointimal hyperplasia, which may lead to the appearance of a new shrinkage and occlusion (typically in the order of 5-20% at 8 months after treatment). The risk of restenosis is linked to an unfavorable early re-shaping process after the intervention, it is a collateral effect of the standard healing process so that, usually, once the risk period has expired (6-8 months after treatment) a good result can be considered definitive. The rate of restenosis after coronary angioplasty is 20-30%, a percentage that decreases dramatically with the implant of coronary stents, which can effectively prevent further occlusions of the coronary arteries. In fact, the results regarding the reduction of restenosis using stents are very different if bare or non-coated stents, the so-called BMS (bare metal stents), are used or if medicated stent, the so-called DES (drug-eluting stent), are used.

The latter have allowed a drastic decrease in cases of restenosis and therefore the need to re-revascularization of 50-70% compared with BMS. In patients with BMS, in-stent restenosis occurs in percentages varying between 12% and 30%, and may require different interventions to re-open the stent. Such interventions may include a new balloon angioplasty, a mechanical demolition of the obstructing tissue, an implant of a new stent within the previous stent, intracoronary radiation (also called brachytherapy), etc..

The DES were introduced in the 2000s and are stents coated with a thin polymer containing a substance with antiproliferative and/or immunosuppressive action that inhibits the proliferation of smooth muscle cells and the neointimal hyperplasia within the stent. These substances are composed of anti-cancer or anti-rejection drugs which are released in a short time or gradually and slowly in a certain number of days, or which after a long initial release phase, can remain in the polymer for an undefined time. Among the currently used drug-eluting stents, particularly important are those coated with paclitaxel, the so-called PES (paclitaxel-eluting stent). Paclitaxel is a chemotherapeutic agent belonging to the group of taxanes and is used for the treatment of various neoplasies.

Paclitaxel interferes with cell migration and proliferation, thus reducing intimal hyperplasia caused by vascular trauma. The drug alters the activity of inflammatory cells that are directly involved in neointimal formation induced by the stent and thanks to its lipophilic and its mode of action results in a lasting antiproliferative effect. Numerous studies have shown that the Paclitaxel shows an antiproliferative effect more effectively than other pharmacological inhibitors of neointimal hyperplasia since, acting directly on intracellular micro-tubules, it permanently favors the formation thereof, and interferes with the cell capacity to maintain its shape and movement direction, to transmit intracellular signals and to carry out intracellular transport.

Randomized studies have shown that DES cause a net decrease in restenosis, thus decreasing the need for new revascularization compared with BMS.

However, the since the metallic structure of the medicated stents remains uncoated, subsequently to the drug release, a stent thrombosis car occur.

The stent thrombosis is a thrombotic implant obstruction, with partial or complete occlusion of the blood flow of the corresponding myocardic area, and thus of a total vessel occlusion and ischemia of the perfused tissue.

The substances that coat DES stents inhibit both "negative" and "positive" cell proliferation; such substances also inhibit stent re-endothelisation (formation on the stent net of a neoendothelium which restores the physiologic functions of the vessel wall) so that the stent metal structure is exposed to the blood flow inside the arteries; such presence is deemed as a foreign body which, without an endothelium, becomes highly thrombogenic.

Therefore the drugs in question prevent endothelial cell re-growth and that of tissues necessary to vessel regeneration; the vessel thus remains uncoated so that a risk occurs that, in such zones, thrombus may be formed.

In order to avoid the discussed complications, new strategies have been adopted with respect traditional balloon angioplasty, thereby creating the balloon catheter coated with drugs and the local drug release balloon catheter.

The use of medicated balloons, the so called DCB (drug-coated balloon) for the prevention of restenosis in coronary and peripheral arteries has received a lot of attention and the first positive results have been reported for paclitaxel-coated balloons.

This method has been tested in several studies which demonstrated a significant reduction in incidence of restenosis compared with conventional balloon. The DCB coated with Paclitaxel are devices with balloons coated with a matrix of paclitaxel that allow a quick transfer of the drug to the tissue of the target lesion. When the balloon is inflated to dilate the narrowed vessel, paclitaxel is released directly into the diseased area by contact. The DCB have a structure to permit their use in coronary territories in which the use of DES is problematic or not particularly effective as small vessels and bifurcations.

The local drug release balloon catheters, the so called DEB (drug-eluting balloons), are a valid alternative to medicated stents or to traditional balloons, since they are very flexible catheters and allow to prevent vascular trauma due to compression and stenting.

The balloons are devices that are temporarily inflated in order to release the drug during a 30 to 60 seconds period inside the vessel walls, and are subsequently removed without any implant remaining; differently, the stents are permanent implants.

DEB allow an homogeneous transfer of drug to the vessel wall, a release of high concentration of the same drug and the absence of a polymer decreases the probability of chronic inflammation.

Several studies have demonstrated the effectiveness of the described devices with the method of infusion of liquid drugs. In fact the balloon catheters for local release of drug currently on the market have various desirable characteristics such as their flexibility that allows its use for the tortuous vessels, their form which facilitates its advancement through the stenosis and their correct positioning with respect to the area to be treated. All these features allow their use for the treatment of the narrowing of the vessels with in stents restenosis and for the treatment of stenosis of small coronary vessels due to atherosclerosis.

Document WO 02/089899 discloses devices, methods, and kits for inhibiting restenosis and hyperplasia after intravascular intervention. In particular, the present invention provides controlled radiosensitizer or immunomodulator delivery in combination with ionizing radiation to selected locations within a patient's vasculature to reduce and/or inhibit restenosis and hyperplasia rates with increased efficacy. In one embodiment, a device includes a proximal end and distal end, an ionizing radiation source coupleable to the catheter body for applying radiation doses to a body lumen, and means coupleable to the catheter body or the radiation source for releasing a radiosensitizer or immunomodulator to the body lumen.

### SUMMARY OF THE INVENTION

The catheters actually on the market to treat vessel with drugs have difficulties to manage the exact drug quantity that is in contact with the vessel and to drain back the drug once the device has been removed; other problems are related to the stop-flow management as very often a blood flow stop for only 30" causes angina.

It is an object of the present invention to provide a device for treatment of a blood vessel as further disclosed in claim 1, which allows the application of a drug to the walls of a determined area of the blood vessel, without the drug being dragged by the blood flow to areas which should not undergo the treatment.

Another object of the present invention is to provide a device that allows the application of a drug to the walls of a determined area of the blood vessel, without any particular time constraints, i.e. which allows a prolonged application of a drug without causing for example hypoxic or ischemic phenomena.

Another object of the present invention is to provide a device that allows to carry out different treatments depending on the drug that must be applied.

A further object of the invention is to provide a device that allows to apply the drug to the zone to be treated without creating overpressure in the vessel and permitting to manage the amount of drug to be used.

Another object of the invention is to provide a device that can be used to treat more than one damaged area or particularly wide damaged areas; as it will become more clear in the following, the device according to the invention can be moved inside the same patient, and thus can be used for different drug treatments, thereby reducing costs with respect to traditional systems.

Another object of the invention is to provide a device that allows to dose and optimize the amount of drug used for the treatment.

### BRIEF DESCRIPTION OF THE DRAWINGS

These and other objects are substantially achieved by a device for treatment of a blood vessel according to the attached claims.

Further features and advantages will become more apparent from the detailed description of a preferred and non exclusive embodiment of the invention. The description is provided hereinafter, with reference to the accompanying drawings, provided by way of non limiting example, wherein:
- Fig. 1 shows a schematic partially exploded perspective view of a device according to the invention, in which some parts have been cancelled in order to highlight other parts;
- Fig. 2 schematically shows the same view of Fig. 1, in which different details are highlighted;
- Fig. 3 shows a larger view of a detail of Fig. 1 and Fig. 2;
- Fig. 4 shows the device of Fig. 1 and Fig. 2 in a different operative state;
- Fig. 5 shows an operating step of the device of Fig. 1, 2 and 4;
- Fig. 6 shows a is a larger view of a detail of the device according to the invention when used in a blood vessel;
- Fig. 7 shows a further operative condition of the device of Fig. 1, 2, 4;
- Fig. 8 shows a detail regarding a further operating condition of the device;
- Figs. 9, 9a, 9b show schematic views of a different embodiment of the invention.

The drawing show different aspects and embodiments of the invention and, where appropriate, structures, components, materials and/or similar elements in different figures are indicated by the same reference numerals.

### DETAILED DESCRIPTION OF THE INVENTION

Referring to the drawings, 1 indicates a device for treatment of a blood vessel according to the present invention.

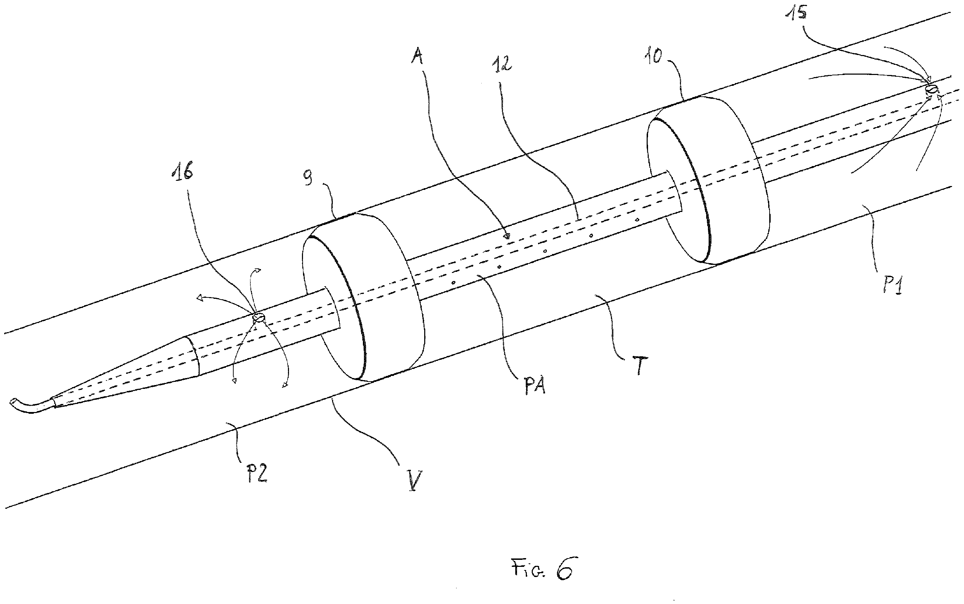

Device 1 comprises first of all a catheter C configured to be inserted in a blood vessel V to be treated (Fig. 6).

Catheter C is preferably used in interventional cardiology procedures, with femoral or radial approach. For example the overall length of catheter C can be about 1000 to 1500 mm.

At a non invasive end 2, the catheter C has a hub to which extensions converge, preferably associated with respective taps. For example the extensions can be three, as will be more clear in the following.

Preferably the catheter C can be of "rapid exchange" type, i.e. able to be quickly removed. Preferably catheter C is adapted to run on a guidewire 5 by means of a respective guide lumen 12', 12.

Preferably the guidewire 5 extends from an invasive end 7 of the catheter C to a lateral exit hole 6, closer to the non invasive end 2.

For example, the lateral exit hole 6 can be located at 250 mm from tip.

Preferably catheter C has a first part 3' extending from the non invasive end 2 that can be extruded, for example. In the preferred embodiment, the first part 3' is substantially cylindrical. Catheter C can have a second part 4.

In the preferred embodiment the second part 4 is substantially cylindrical.

Preferably the first part 3' has a diameter shorter than the second part 4. The first part 3' can have a diameter of 0,5 to 1,5 mm, whereas the second part 4 can have a diameter of 1 to 3 mm. For the coronary treatment the diameter of the first and second part 3', 4 is comprised between 1 and 1,4 mm.

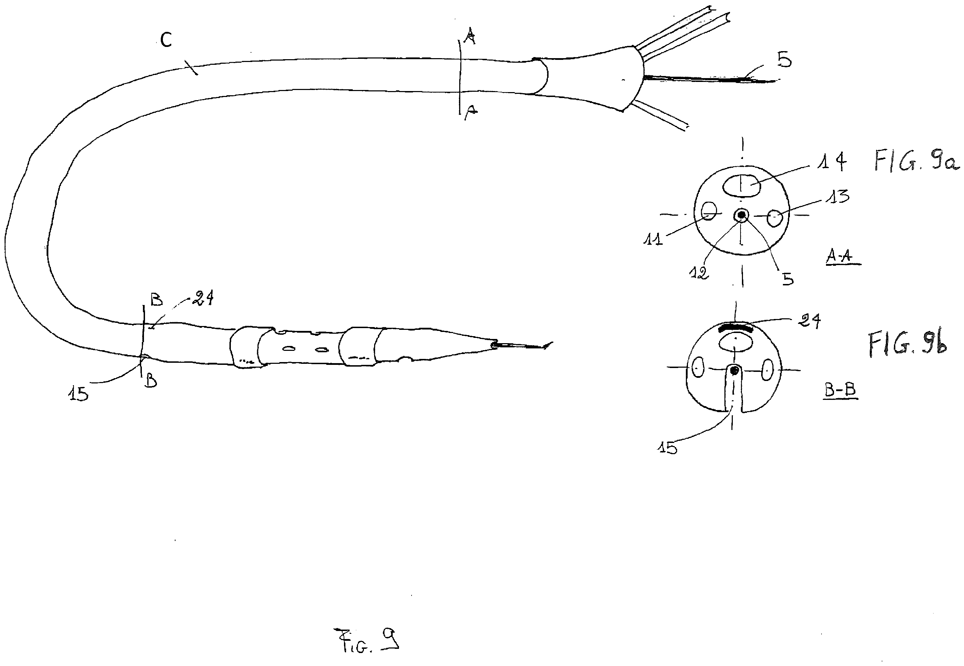

In a different embodiment, schematically shown in Fig. 9, the first and second parts 3', 4 can have the substantially same diameter.

Catheter C can be made by polyamide, polyurethane, polyethylene or any thermoplastic materials suitable for medical use.

Preferably the compatibility of the material with the delivered drug is previously verified. Preferably catheter C has a third part 3", extending from an end of the second part 4 to the invasive end 7.

The third part 3" preferably is cylindrical and its diameter in cross section is substantially the same as the diameter of the second part 4.

The mentioned invasive end 7 has preferably a centered lumen 8 which houses the guidewire 5. Preferably the invasive end 7 has a tapered shape ending with a centered hole corresponding to centered lumen 8 where the guidewire 5 is positioned. Advantageously the tip of the invasive end 7 is of the atraumatic type, i.e. very tapered and soft. For example, it can be over molded on the catheter C body.

According to the invention the device 1 further comprises a selection member S associated to the catheter C to prevent a blood flow in at least a determined section T of the blood vessel V.

In practice, when the device 1 is employed, and the catheter C is inserted in the blood vessel V, the selection member S prevents blood from flowing in the determined section T of the same vessel V (i.e. the section on which the intervention must be made). In this way it is possible to effectively intervene on the determined section T, for example, as it will become more clear in the following, by means of insertion of a suitable drug.

The selection member S is mounted on the catheter C so as to define on the same catheter an active portion PA which, in use, is located at the determined section T of the blood vessel V. The drug is applied to a wall of the determined section T by means of the active portion PA. Preferably the selection member S is located at the third part 3" of the catheter C. The selection member S comprises a first and a second inflatable elements 9, 10. Preferably the first and second inflatable element 9, 10 radially extend from the outer surface of the catheter C toward the space outside the catheter C.

Preferably the first and second inflatable element 9, 10 are fixed to the outer surface of catheter C, for example by gluing.

Preferably the first and second inflatable element 9, 10 are axially spaced from each other along the longitudinal extension of catheter C. The determined section T of the blood vessel V is longitudinally delimited by the first and second inflatable elements 9, 10.

In practice the determined section T extends along the longitudinal extension of the blood vessel V between the first inflatable element 9 and the second inflatable element 10. The active portion PA of catheter C is interposed between the first inflatable element 9 and the second inflatable element 10.

In other terms the active portion PA extends along the longitudinal extension of catheter C between the first inflatable element 9 and the second inflatable element 10.

Preferably the first inflatable element 9 is located close to the invasive end 7 of the catheter C and the second inflatable element 10 is located at a distance comprised between 10 and 70 mm from the first inflatable element 9.

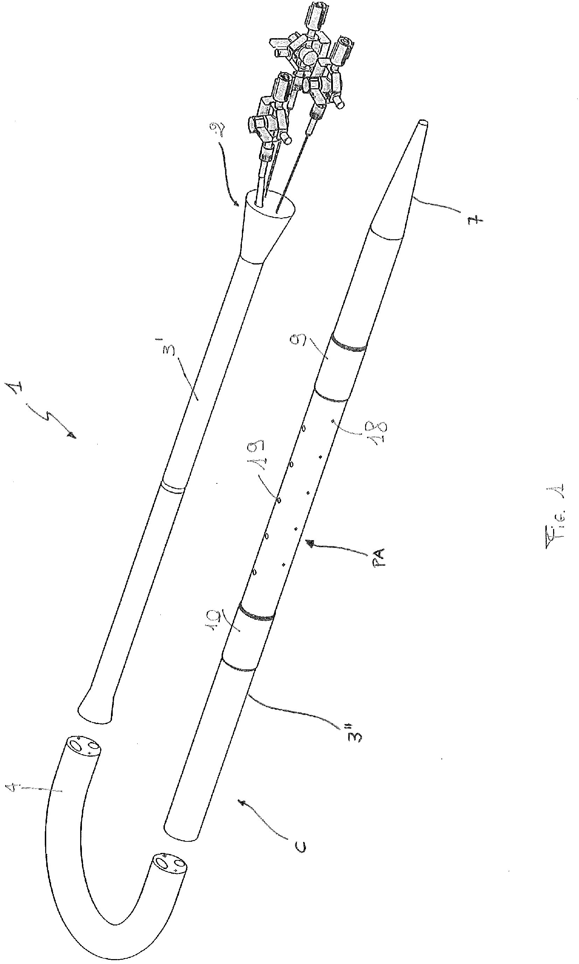

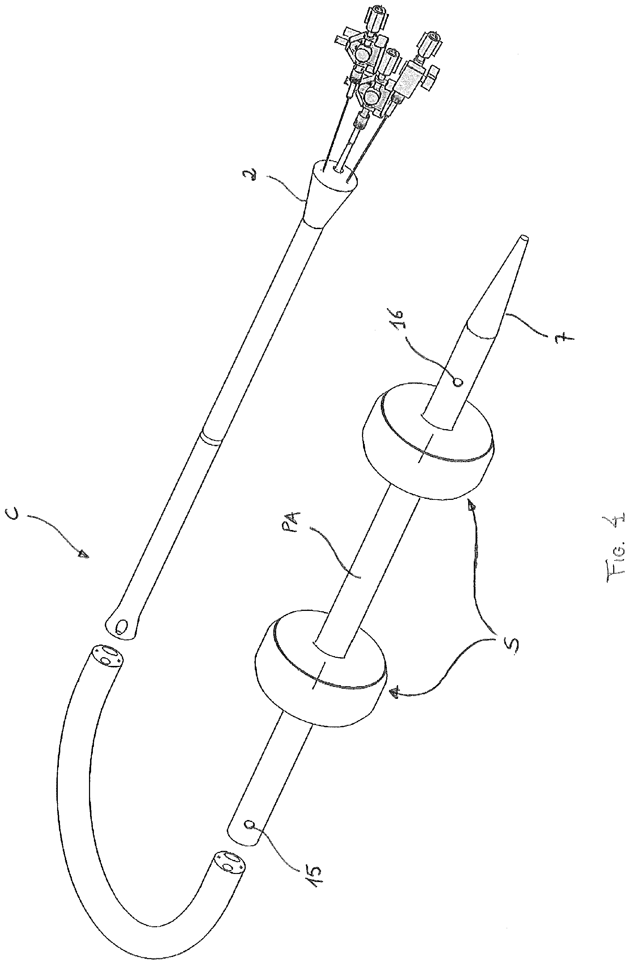

In rest condition the first and the second inflatable elements 9, 10 are deflated (Fig. 1, 2, 5, 9). In working condition, when the selection member S has to insulate the determined section T, the first and second inflatable element 9, 10 are inflated (Fig. 4, 6, 7, 8).

When the first and the second elements 9, 10 are inflated, they adhere to blood vessel V inner wall preventing the blood in the vessel V upstream and downstream the selection member S to flow to/from the determined section T.

The first and the second inflatable elements 9, 10 can be inflated up to a suitable diameter, for example between 2 and 5 mm.

In practice the first and the second inflatable elements 9, 10 can be realized in form of balloons, as shown in the annex drawings. The balloons can be compliant or non compliant. Preferably the balloons support a low inflation pressure.

Preferably the balloons are soft, made of elastomeric material and atmospheric pressure inflatable. The material they are made of is verified for compatibility with the drug to be used. For example a suitable material can be Poly-Isoprene.

Advantageously since the inflation pressure is very low, the pressure exerted by the balloon against the vessel V inner wall is not damaging it.

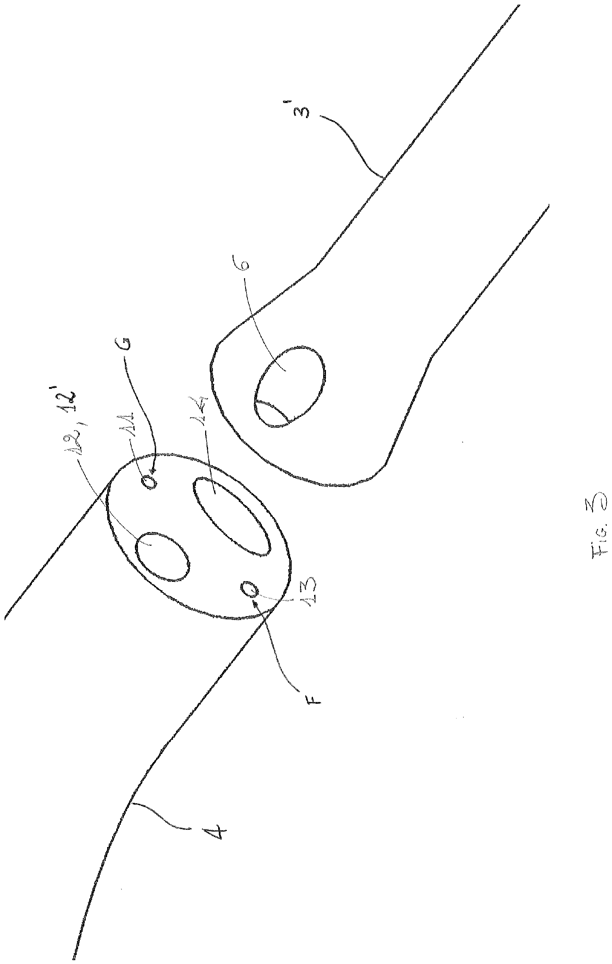

Preferably the device 1 also comprises an inflation system G (Fig. 3) to inflate first and second elements 9, 10.

Preferably the inflatable elements 9, 10 can be inflated substantially simultaneously or independently from each other.

The fact that, for example, the first inflatable element 9 can be inflated without inflating, at the same time, the second inflatable element 10, allows to perform preliminary tests on the determined section T, so as to evaluate possible problems o possible complications before isolating the determined section T.

By way of example, by means of a suitable contrast fluid, it is possible to verify the presence of possible branches of the blood vessel V afferent to the determined section T which might cause undesired flows of drug from the determined section T during treatment.

In more detail, the inflation system G comprises one or more inflation lumens 11 extending along catheter C to connect the first and second inflatable elements 9 and 10 to a source of inflation fluid.

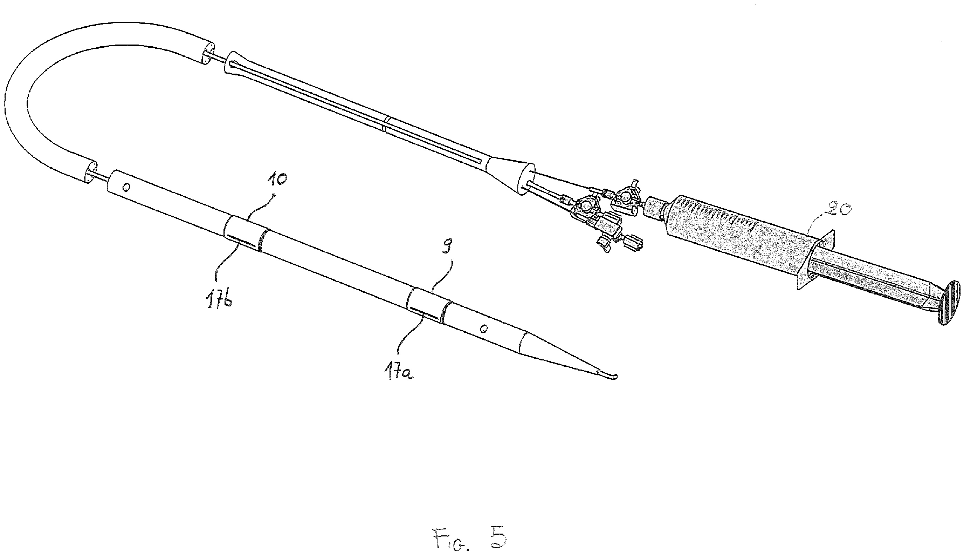

The source of inflation fluid is identified by way of example with a syringe 20 in Fig. 5.

Should and independent inflation be provided, each inflatable element 9, 10 is connected with a respective independent lumen to the source of inflation fluid.

The inflation fluid can be for example a physiologic solution or a physiologic solution combined with a contrast fluid.

In this way it is possible to significantly increase the likelihood that the inflation is performed without bubbles being formed or inserted in inflatable elements 9, 10.

According to the invention, device 1 also includes self-perfusion means A (Fig. 6) associated to catheter C.

The self-perfusion means A allow fluidic communication between a first portion P1 of the blood vessel V upstream the determined section T with a second portion P2 of the same vessel V downstream the determined section T.

Preferably the self-perfusion means A comprise at least a self-perfusion lumen 12 extending inside the catheter C; said self-perfusion lumen 12 connects the first portion P1 with the second portion P2 of blood vessel V.

The term self-perfusion indicates the blood flowing from upstream to downstream the zone in which the blood does not flow, i.e. the zone isolated by the selection member S.

The self-perfusion lumen 12 is preferably associated to a first hole 15 located upstream the selection member S, for example at a distance included between 60 and 100 mm from the tip of the catheter C, and a second hole 16 located downstream the selection member S.

In other embodiments of the invention that are not shown but belong anyway to the present invention, it is possible to provide a different number of holes upstream and/or downstream the selection member S.

According to the invention, device 1 also comprises a treatment system F (Fig. 3) that is associated to catheter C and that lets flow a fluid drug trough catheter C. This drug is made flow at least until the determined section T in order to intervene on the designated area to be treated. Advantageously the treatment system F also allows to recover the drug during and/or after treatment.

Preferably the treatment system F defines, in cooperation with the determined section T of blood vessel V, and possible syringes or similar member associated to the same treatment system F, a closed hydraulic system.

This closed system, according to the present invention, is totally under control, so that circulation of fluids (blood, saline, drugs, etc.) within said closed system is regulated according to the designated treatment. The treatment system F includes one or more treatment lumina 13 extending inside catheter C and ending in one or more holes 18 located at the active portion PA to deliver the drug.

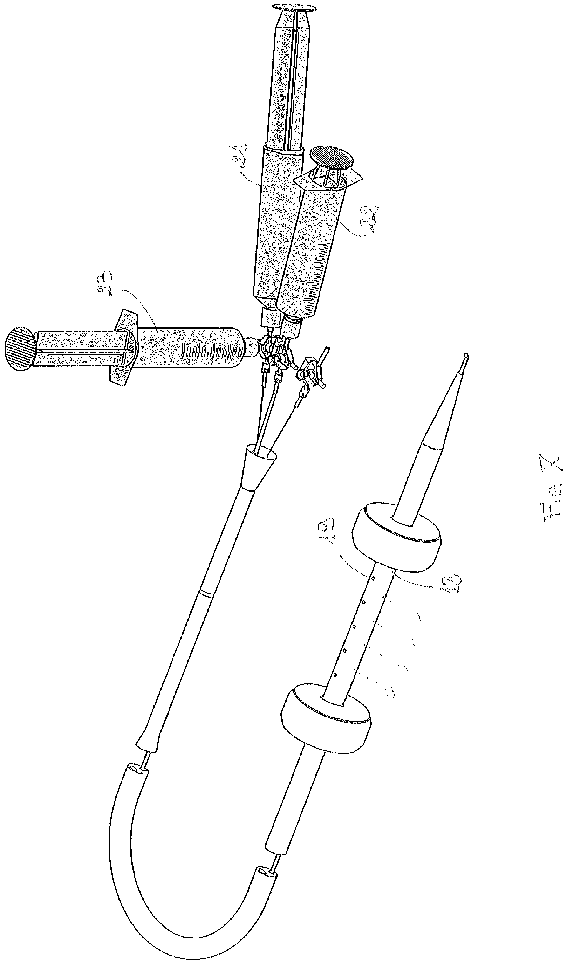

Treatment lumen 13 ends at the non invasive end of catheter C, where it can be connected to a respective syringe 21 (Fig. 7), or equivalent member, for drug delivery.

Advantageously, said one or more treatment lumina 13 and said one or more holes 18 allow said fluid drug to come to contact with the determined section T of the blood vessel V.

In other terms said one or more treatment lumina 13 and said one or more holes 18 are sufficient in order to let the drug propagate in the determined section T to directly reach the radially inner surface of the same section, once the same drug has exited from hole(s) 18, so that the treatment can be performed.

This means that the drug does not need further conduits or structures to reach the inner surface of the determined section T.

In particular the device 1 does not comprise any membranes or transfer supports of said drug interposed between said one or more holes 18 and the inner walls of the determined section T. In this regard it is to be noted that preferably the outer diameter of the device 1, at the active portion PA, remains substantially unchanged during working of the same device 1.

In particular, the delivery to the inner surface of the determined section T of the fluid drug occurs without the outer diameter of the device 1, at the active portion PA, undergoes substantial modifications (e.g. increases).

The fluid drug delivered by means of hole(s) 18 can thus propagate inside the whole volume delimited by the first and second inflatable elements 9, 10 and by the inner surface of the determined section T.

The device 1 further comprises one or more return lumina 14 (Fig. 3) afferent to the active portion PA through one or more holes 19, in order at least to drain blood from the determined section T when the drug is injected in the same determined section T.

In other terms, when drug is injected in the determined section T, previously existing blood flows outside through lumen 14, so as to prevent undue pressure increases in the zone and to allow drug to effectively act on the walls to be treated.

Analogously, at the end of the treatment, the drug can be completely removed by suction and "clean" blood can be inserted, that was withdrawn from the determined section T at the beginning of the procedure.

The mentioned closed system is thus realized by means of the treatment lumen 13, the return lumen 14; such system can conveniently also include one or more syringes or other equivalent devices in order to promote the flow of the employed fluids.

The closed system used to withdraw blood at the beginning of the procedure, to inject and possibly make flow the sole drug during treatment, to withdraw the drug at the end of the treatment, and to re-inject the blood initially withdrawn, allows to achieve the advantage not to have residues of drug in the determined section T at the end of the procedure. It is to be noted that, by means of such system, it is possible to carry out both treatments that require a static application of the drug to the walls of the determined section T for a certain time interval, and treatments that require a continuous flow entering into and exiting from the determined section T of the drug to be applied.

The selection of the treatment to be executed can be made without particular constraints due to the great employment flexibility of the device 1 according to the invention.

Preferably the device 1 comprises at least one injection element and at least one withdrawal element, associated with said one or more treatment lumina 13 and said one or more return lumina 14 respectively, to continuously inject and withdraw said drug into/from said determined section T.

In this way a continuous treatment is carried out by means of said drug.

By means of the withdrawal element it is also possible to recover the drug during and/or at the end of the treatment.

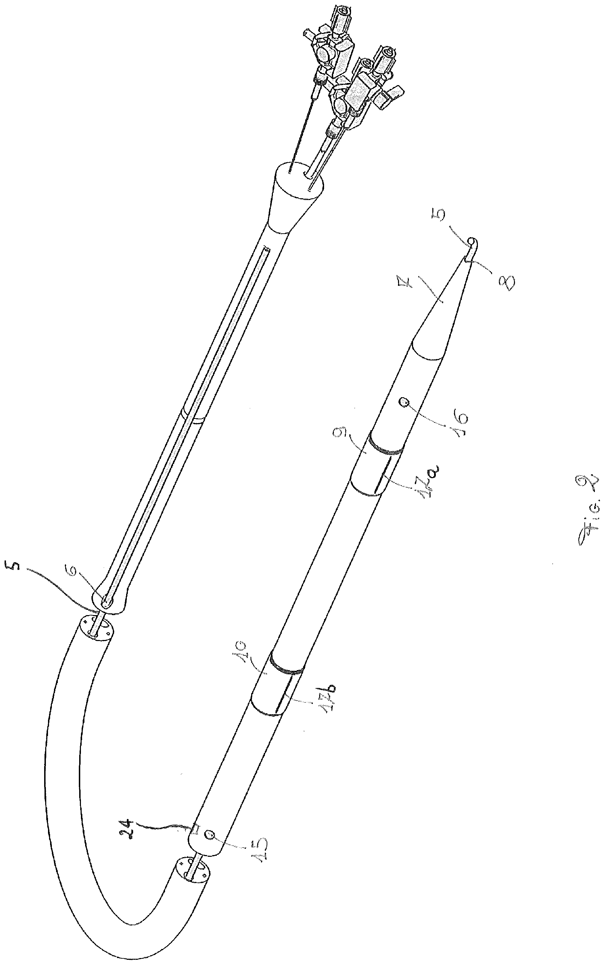

The device 1 preferably comprises one or more markers 17a, 17b, 24 (Fig. 2), which can be located at one or more of the following elements:
- an end of the self-perfusion lumen 12 located upstream the determined section T, i.e. the mentioned first self-perfusion hole 15 (marker 24);
- the first inflatable element 9 (marker 17a);
- the second inflatable element 10 (marker 17b).

Preferably the mentioned markers 17a, 17b, 24 can be of the radiopaque type, so as to be detected in radioscopy during the insertion of catheter C.

It is to be noted that marker 24 is advantageous in order to indicate till where it is suggested to retract guidewire 5 from the tip without the risk of losing it when lumina 12, 12' (disclosed hereinafter) coincide.

Preferably catheter C comprises a guide lumen 12' (Fig. 3) configured to house a guidewire 5 of device 1.

In practice, in lumen 12' a guidewire 5 is inserted on which catheter C can run when the device 1 is used.

In the preferred embodiment guide lumen 12' and self-perfusion lumen 12 coincide at least partially.

In this way it is possible to use one single lumen for housing the guidewire 5 and for the blood flow used for self-perfusion.

It is to be noted that self-perfusion lumen 12 (which coincides, as said, with guide lumen 12') and guidewire 5 are suitably shaped and dimensioned so that guidewire 5 does not completely obstruct the lumen and permits self-perfusion also when it is inserted in the same lumen.

As schematically shown in Fig. 2, holes 6, 8 for guidewire 5 and holes 15, 16 for self-perfusion are preferably formed on the same lumen.

It is to be noted that the self-perfusion holes are suitably shaped so as not to be used for the guidewire.

For example, it is hereinafter described the functioning of device 1 in an application in which it is used for a coronary treatment, i.e. to treat by means of a drug a stenotic area of a coronary artery.

Catheter C is inserted in the selected coronary from femoral (or radial) artery using standard Seldinger technique.

Catheter is placed under scopic control preferably considering as references the above mentioned markers 17a, 17b, 24.

The invasive end of catheter C is positioned so that the selection member S is properly located with respect to the area to be treated, i.e. the determined section T.

In this scenario, the determined section T is interposed between the first and second inflatable element 9, 10. In practice, active portion PA of device 1 is positioned at the determined section T which has to be treated.

The inflatable elements 9, 10 are the inflated using for example a suitable syringe which injects in the same saline or saline mixed with contrast fluid.

The inflatable elements 9, 10 are inflated till they adhere substantially tightly to the inner walls of the blood vessel in which catheter C is inserted.

Now the stenotic coronary tract that has to be treated is isolated and no more perfused by blood. The portion downstream the selection member S and in particular downstream the first inflatable element 9 is still perfused by means of self-perfusion means A.

Finite elements analysis and in vitro tests demonstrated that self-perfusion means A is effective even with guidewire in place. When guidewire 5 is retracted from self-perfusion lumen 12, i.e. when placing the guidewire 5 upstream the first self-perfusion hole 15, and maintaining it downstream its exit hole 6, renders the self-perfusion blood flow more linear, i.e. without even slightly turbulent zone.

A detailed analysis has shown that self-perfusion is very effective (i.e. it guarantees a perfusion of about 200 ml/min of blood) if the guidewire is retired from its position till upstream the hole 15 in which blood enters.

The same analysis has shown that self-perfusion is still effective if the guidewire is maintained *in situ.*

In this latter case, the blood flow enters a wider turbulent region which does not affect the stability of the hemodynamic condition.

Self-perfusion using aortic blood permits long treatment time, so unstable patients can be treated. It is to be noted that other treatments based on total occlusive techniques resulted in ischemic or fibrillation complications.

In another embodiment, second self-perfusion hole 16 can be not realized, since blood can exit even only from the hole located at the invasive end of catheter C. In other terms, the first self-perfusion hole lets the blood flow from the first portion P1 till the outlet of centered lumen 8. The area to be treated is now isolated and ready to be treated.

A syringe 21 containing drug is connected with treatment lumen 13 so that the drug is infused in the coronary artery at the determined section T through one or more holes 18 located at the active portion PA of catheter C.

Preferably the drug is an anti-proliferative liquid drug. The drug can now act, i.e. settle on the wall cells and inhibit proliferation thereof.

In order to avoid undesired overpressure in the coronary artery, the system allows the creation of a low pressure closed system. Thus, as said, it is envisaged the use of one or more return lumina 14 which connect the determined section T with an empty vacuum syringe 22 (Fig. 7): the blood replaced by infused drug is pushed through holes 19 and passively collected in syringe 22.

When the volume delimited by the selection member S is full of drug it is necessary to let it act for a suitable time. Coronary vessel is anyway self perfused, so that there is no need to speed up the process.

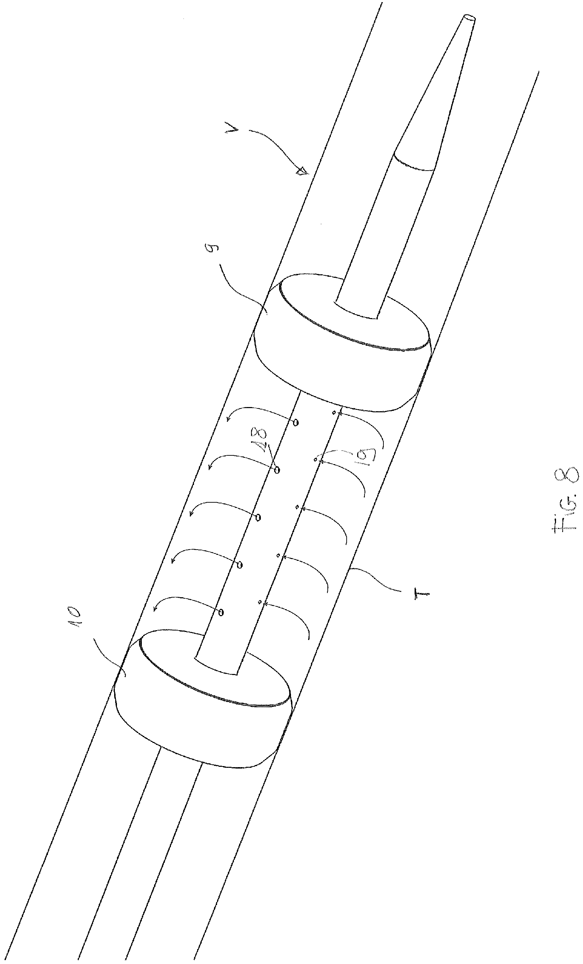

At the end of the drug contact time, a saline solution can be infused in the determined section T by syringe 23 (Fig. 7, 8) through holes 18, while drug is passively or forcedly drained through holes 19 towards syringe 22.

Once the volume of drug has been replaced with the saline solution, the operator is certain that in the coronary there are no residues of drug which may inadvertently be transported in the blood flow. In this regard, in vitro tests demonstrated the capability of the selection member to isolate the coronary section to be treated and the possibility to drain the entire drug volume with no vessel depression or coronary lesions.

In order to replace the volume of the determined section T isolated by the selection member S with blood and/or saline solution, the roles of the injection and suction lumina can be inverted, as well those of holes of connection with the treatment area. In other words, the lumen previously used to withdraw blood initially present in the determined section T can be subsequently used to inject blood and/or physiologic solution at the end of the treatment, whereas the lumen used for drug infusion can be used to withdraw the same drug.

At the end of the procedure the first and second inflatable elements are deflated and subsequently the catheter C is removed.

It is to be noted that device 1 according to the invention is intended for liquid or soluble specialist drugs which, when in contact with the vessel walls, can treat or prevent certain pathologies thereof.

This operation is advantageously executed when it is necessary to isolate the drug from the continuous blood flow for a certain time period, whereas the vessel is anyway perfused due to the structure of device 1.

It is also to be noted that the device disclosed and shown is of the "monorail" type, but the basic features thereof can also be adapted to an "over the wire" model, by applying suitable geometric modifications.

If the catheter is of the "over the wire" type, the guidewire is centered from the tip to the outer part of the catheter, thus the cross section presents four lumina, as shown in figures 9, 9a, 9b, wherein the catheter C has a substantially constant diameter.

The device herein disclosed is intended for use in coronary in order to treat specific stenotic areas, and allows to achieve the above indicates aims.

Such device, having suitable dimensions, can be employed also in other vessels, for instance peripheral vessels, or in other body areas where it is necessary to realize a closed infusion system, for a continuous or calibrated infusion, and subsequent removal or washing, of a liquid or suspension or emulsion drug.

Therefore, in summary, the method for treatment of a blood vessel that is carried out comprises the following steps:
- isolating a determined section T of a blood vessel V, so that the blood that flows upstream and/or downstream said determined section T does not enter/exit from the determined section T;
- connect the vessel portion upstream the determined section T with the vessel portion downstream the determined section T, so that the blood coming from the portion upstream the determined section T can flow in the portion downstream the determined section T;
- infuse a drug in the determined section T.
Preferably the method further comprises a step of progressively withdrawing the blood from the determined section T while the drug is infused.

Preferably, in a subsequent moment, a step of withdrawing the drug previously infused is performed.

Preferably, while the drug is withdrawn, a step of infusing a substitute fluid, comprising form example physiologic solution, into the determined section, is carried out.

It is to be noted that the steps indicated hereabove are preferably carried out while the determined section T is isolated as said above.

Preferably the method comprises a final step in which the isolation of the determined section T is removed. In this way the blood flowing in the portion upstream the determined section T can flow again in the determined section T before flowing in the portion downstream the determined section T.

## Claims

1. A device for treating a blood vessel, comprising:
a catheter (C) adapted to be inserted into a blood vessel (V) to be treated;
a selection member (S) associated with said catheter (C) to prevent a blood flow in at least one given section (T) of said blood vessel (V), wherein said selection member (S) comprises a first inflatable element (9) and a second inflatable element (10), said given section (T) being delimited longitudinally by said first and second inflatable elements (9, 10);
a self-perfusion means (A) associated with said catheter (C) to put a first portion (P1) of said blood vessel (V) upstream of said given section (T) in fluid communication with a second portion (P2) of said blood vessel (V) downstream of said given section (T);
a treatment system (F) associated with said catheter (C) to cause a drug in fluidic state to flow, through said catheter (C), at least down to said given section (T)
wherein said selection member (S) is mounted on said catheter (C) so as to delimit on said catheter (C) an active portion (PA) adapted to radially face said given section (T) of said blood vessel (V),
wherein said treatment system (F) comprises one or more treatment lumina (13) extending within said catheter (C) and ending in a plurality of treatment holes (18) at said active portion (PA) to deliver said drug,
wherein said one or more treatment lumina (13) and said plurality of treatment holes (18) allow said drug in fluidic state to come to contact with said given section (T); and
one or more return lumina (14) starting in a plurality of return holes (19) at said active portion (PA) and extending within said catheter (C) to allow at least a blood outflow from said given section (T) when said drug is delivered into said given section (T) and, after a treatment, a drug outflow from said given section (T) and re-introduction of the blood previously withdrawn from said given section (T).

2. A device according to claim 1 wherein said device does not comprise membranes or transferring supports for said drug interposed between said plurality of treatment holes (18) and an inner surface of said given section (T).

3. A device according to claim 1 or 2, wherein said treatment system (F) also allows recovering said drug during and/or at the end of the treatment and to re-inject blood previously withdrawn from said given section (T).

4. A device according to any one of the preceding claims, wherein said treatment system (F) defines, in cooperation with at least said given section (T), a closed hydraulic system.

5. A device according to any one of the preceding claims, wherein the active portion (PA) of said catheter (C) is interposed between said first and second inflatable elements (9, 10).

6. A device according to any one of the preceding claims, further comprising an inflation system (G) to inflate, in a substantially simultaneous or selective manner, said first and second inflatable elements (9, 10).

7. A device according to claim 6, wherein said inflation system (G) comprises one or more inflation lumina (11) extending along said catheter (C) to connect said first and second inflatable elements (9, 10) to an inflation fluid source, wherein each of said inflatable elements (9, 10) is connected to said inflation fluid source through a respective independent lumen.

8. A device according to any one of the preceding claims, wherein said self-perfusion means (A) comprise at least one self-perfusion lumen (12) extending within said catheter (C) and joining said first and second portions (P1, P2) of said blood vessel (V) at a first self-perfusion hole (15) and a second self-perfusion hole (16), respectively, wherein said first and second self-perfusion holes (15, 16) are shaped so as not to be used for a guidewire (5).

9. A device according to claim 1 further comprising at least one injection member and at least a withdrawal member, associated with said one or more treatment lumina (13) and said one or more return lumina (14) respectively to continuously inject and withdraw drug into/from said given section (T).

10. A device according to any one of the preceding claims, further comprising one or more markers (17, 24), preferably of the radiopaque type, in particular positioned on one or more of the following elements:
one end of said self-perfusion lumen (12) located upstream of said given section (T);
said first inflatable element (9);
said second inflatable element (10).

11. A device according to any one of the preceding claims, wherein said catheter (C) further comprises a guide lumen (12') for housing a guidewire (5) of said device (1).

12. A device according to claim 10, wherein said guide lumen (12') coincides at least partially with said self-perfusion lumen (12).

## Patentansprüche

1. Vorrichtung zum Behandeln eines Blutgefäßes, umfassend:
einen Katheter (C), der angepasst ist, in ein Blutgefäß (V), das behandelt werden soll, eingeführt zu werden;
ein Selektionsteil (S), das mit dem Katheter (C) verbunden ist, um einen Blutfluss in mindestens eine gegebene Sektion (T) des Blutgefäßes (V) zu verhindern, wobei der Selektionsteil (S) ein erstes aufblähbares Element (9) und ein zweites aufblähbares Element (10) umfasst, wobei die gegebene Sektion (T) in Längsrichtung durch das erste und zweite aufblähbare Element (9, 10) begrenzbar ist;
ein Selbstperfusionsmittel (A), das mit dem Katheter (C) verbunden ist, um einen ersten Teil (P1) des Blutgefäßes (V) stromaufwärts der gegebenen Sektion (T) in fluidischer Verbindung mit einem zweiten Teil (P2) des Blutgefäßes (V) stromabwärts der gegebenen Sektion (T) zu verbinden;
ein Behandlungssystem (F), das mit dem Katheter (C) verbunden ist, um ein Arzneimittel in fluidischem Zustand zu veranlassen, durch den Katheter (C) hinunter zu der gegebenen Sektion (T) zu fließen,
wobei der Selektionsteil (S) so auf dem Katheter (C) angebracht ist, um auf dem Katheter (C) einen aktiven Bereich (PA) zu begrenzen, der eingerichtet ist, der gegebenen Sektion (T) des Blutgefäßes (V) radial gegenüber zu liegen,
wobei das Behandlungssystem (F) ein oder mehrere Behandlungslumina (13) umfasst, die sich innerhalb des Katheters (C) erstrecken und in mehreren Behandlungslöchern (18) in dem aktiven Bereich (PA) enden, um das Arzneimittel zu liefern,
wobei das eine oder die mehreren Behandlungslumina (13) und die mehreren Behandlungslöcher (18) es dem Arzneimittel erlauben, in fluidischem Zustand in Kontakt mit der gegebenen Sektion (T) zu gelangen; und
ein oder mehrere Rücklumina (14), die in mehreren Rücklöchern (19) in dem aktiven Bereich (PA) beginnen und sich innerhalb des Katheters (C) erstrecken, um mindestens einen Blutausfluss aus der gegebenen Sektion (T) zu erlauben, wenn das Arzneimittel in die gegebene Sektion (T) geliefert wird, und um nach der Behandlung einen Arzneimittelausfluss von der gegebenen Sektion (T) und Wiedereinführen des Bluts, das zuvor von der gegebenen Sektion (T) entnommen wurde, zu ermöglichen.

2. Vorrichtung nach Anspruch 1, wobei die Vorrichtung keine Membranen oder Transferunterstützungen für das Arzneimittel, das zwischen die mehreren Behandlungslöcher (18) und eine innere Oberfläche der gegebenen Sektion (T) eingefügt wurde, umfasst.

3. Vorrichtung nach Anspruch 1 oder 2, wobei das Behandlungssystem (F) es auch erlaubt, das Arzneimittel während und/oder am Ende der Behandlung zurückzugewinnen und zum Wiedereinführen des Bluts, das zuvor von der gegebenen Sektion (T) entnommen wurde, wieder zu injizieren.

4. Vorrichtung nach einem der vorhergehenden Ansprüche, wobei das Behandlungssystem (F) in Verbindung mit zumindest der gegebenen Sektion (T) ein geschlossenes hydraulisches System definiert.

5. Vorrichtung nach einem der vorhergehenden Ansprüche, wobei der aktive Bereich (PA) des Katheters (C) zwischen dem ersten und dem zweiten aufblähbaren Element (9, 10) zwischengeschaltet ist.

6. Vorrichtung nach einem der vorhergehenden Ansprüche, ferner umfassend ein Aufblähsystem (G) zum Aufblähen in im Wesentlichen gleichzeitiger oder selektiver Weise des ersten und zweiten aufblähbaren Elements (9, 10).

7. Vorrichtung nach Anspruch 6, wobei das Aufblähsystem (G) ein oder mehrere Aufblählumina (11) umfasst, die entlang dem Katheter (C) verlaufen, um das erste und zweite aufblähbare Element (9, 10) mit einer Aufblähfluidquelle zu verbinden, wobei jedes der aufblähbaren Elemente (9, 10) mit der Aufblähfluidquelle jeweils durch ein entsprechendes unabhängiges Lumen verbunden ist.

8. Vorrichtung nach einem der vorhergehenden Ansprüche, wobei die Selbstperfusionsmittel (A) mindestens ein Selbstperfusionslumen (12) umfassen, das sich innerhalb des Katheters (C) erstreckt und das mit dem ersten und zweiten Bereich (P1, P2) des Blutgefäßes (V) an einem entsprechenden ersten Selbstperfusionsloch (15) und einem zweiten Selbstperfusionsloch (16) verbunden ist, wobei das erste und zweite Selbstperfusionsloch (15, 16) geformt ist, um nicht mit einem Führungsdraht (5) verwendet zu werden.

9. Vorrichtung nach Anspruch 1, ferner umfassend mindestens ein Injektionsteil und mindestens ein Zurücknahmeteil, die mit dem einen oder mehreren Behandlungslumina (13) und dem einen oder mehreren Rücklumina (14) entsprechend verbunden sind, um kontinuierlich in/von der gegebenen Sektion (T) Arzneimittel zu injizieren und zurückzunehmen.

10. Vorrichtung nach einem der vorhergehenden Ansprüche, ferner umfassend ein oder mehrere Marker (17, 24), vorzugsweise eines röntgendichten Typs, insbesondere positioniert auf einem oder mehreren der folgenden Elemente:
einem Ende des Selbstperfusionslumens (12), das stromaufwärts der gegebenen Sektion (T) angeordnet ist;
dem ersten aufblähbaren Element (9);
dem zweiten aufblähbaren Element (10).

11. Vorrichtung nach einem der vorhergehenden Ansprüche, wobei der Katheter (C) ferner ein Führungslumen (12') zum Beherbergen eines Führungsdrahts (5) der Vorrichtung (1) umfasst.

12. Vorrichtung nach Anspruch 10, wobei das Führungslumen (12') zumindest teilweise mit dem Selbstperfusionslumen (12) zusammentrifft.

## Revendications

1. Dispositif de traitement d'un vaisseau sanguin, comprenant :
un cathéter (C) adapté pour être inséré dans un vaisseau sanguin (V) à traiter ;
un organe de sélection (S) associé audit cathéter (C) pour empêcher un écoulement de sang dans au moins une section donnée (T) dudit vaisseau sanguin (V), dans lequel ledit organe de sélection (S) comprend un premier élément gonflable (9) et un second élément gonflable (10), ladite section donnée (T) étant délimitée longitudinalement par lesdits premier et second éléments gonflables (9, 10) ;
un moyen d'auto-perfusion (A) associé audit cathéter (C) pour mettre une première portion (P1) dudit vaisseau sanguin (V) en amont de ladite section donnée (T) en communication fluidique avec une seconde portion (P2) dudit vaisseau sanguin (V) en aval de ladite section donnée (T) ;
un système de traitement (F) associé audit cathéter (C) pour amener un médicament dans un état fluidique à s'écouler, à travers ledit cathéter (C), au moins jusqu'à ladite section donnée (T)
dans lequel ledit organe de sélection (S) est monté sur ledit cathéter (C) de manière à délimiter sur ledit cathéter (C) une portion active (PA) adaptée pour faire face radialement à ladite section donnée (T) dudit vaisseau sanguin (V),
dans lequel ledit système de traitement (F) comprend une ou plusieurs lumières de traitement (13) s'étendant au sein dudit cathéter (C) et se terminant dans une pluralité de trous de traitement (18) au niveau de ladite portion active (PA) pour délivrer ledit médicament,
dans lequel lesdites une ou plusieurs lumières de traitement (13) et ladite pluralité de trous de traitement (18) permettent audit médicament à l'état fluidique de venir en contact avec ladite section donnée (T) ; et
une ou plusieurs lumières de retour (14) commençant en une pluralité de trous de retour (19) au niveau de ladite portion active (PA) et s'étendant au sein dudit cathéter (C) pour permettre au moins un écoulement sortant de sang depuis ladite section donnée (T) lorsque ledit médicament est délivré dans ladite section donnée (T) et, après un traitement, un écoulement sortant de médicament depuis ladite section donnée (T) et une réintroduction du sang préalablement prélevé de ladite section donnée (T).

2. Dispositif selon la revendication 1, dans lequel ledit dispositif ne comprend pas de membranes ou de supports de transfert pour ledit médicament interposé entre ladite pluralité de trous de traitement (18) et une surface interne de ladite section donnée (T).

3. Dispositif selon la revendication 1 ou 2, dans lequel ledit système de traitement (F) permet également de récupérer ledit médicament pendant et/ou à la fin du traitement et de réinjecter du sang préalablement prélevé de ladite section donnée (T).

4. Dispositif selon l'une quelconque des revendications précédentes, dans lequel ledit système de traitement (F) définit, en coopération avec au moins ladite section donnée (T), un système hydraulique fermé.

5. Dispositif selon l'une quelconque des revendications précédentes, dans lequel la portion active (PA) dudit cathéter (C) est interposée entre lesdits premier et second éléments gonflables (9, 10).

6. Dispositif selon l'une quelconque des revendications précédentes, comprenant en outre un système de gonflage (G) pour gonfler, de manière sensiblement simultanée ou sélective, lesdits premier et second éléments gonflables (9, 10).

7. Dispositif selon la revendication 6, dans lequel ledit système de gonflage (G) comprend une ou plusieurs lumières de gonflage (11) s'étendant le long dudit cathéter (C) pour raccorder lesdits premier et second éléments gonflables (9, 10) à une source de fluide de gonflage, dans lequel chacun desdits premier et second éléments gonflables (9, 10) est raccordé à ladite source de fluide de gonflage par l'intermédiaire d'une lumière indépendante respective.

8. Dispositif selon l'une quelconque des revendications précédentes, dans lequel ledit moyen d'auto-perfusion (A) comprend au moins une lumière d'auto-perfusion (12) s'étendant au sein dudit cathéter (C) et réunissant lesdites première et seconde portions (P1, P2) dudit vaisseau sanguin (V) au niveau d'un premier trou d'auto-perfusion (15) et d'un second trou d'auto-perfusion (16), respectivement, dans lequel lesdits premier et second trous d'auto-perfusion (15, 16) sont dimensionnés de manière à ne pas être utilisés pour un fil-guide (5).

9. Dispositif selon la revendication 1, comprenant en outre au moins un organe d'injection et au moins un organe de prélèvement, associés auxdites une ou plusieurs lumières de traitement (13) et auxdites une ou plusieurs lumières de retour (14) respectivement pour injecter et prélever en continu du médicament dans/depuis ladite section donnée (T).

10. Dispositif selon l'une quelconque des revendications précédentes, comprenant en outre un ou plusieurs marqueurs (17, 24), de préférence du type radioopaque, en particulier positionnés sur un ou plusieurs des éléments suivants :
une extrémité de ladite lumière d'auto-perfusion (12) située en amont de ladite section donnée (T) ;
ledit premier élément gonflable (9) ;
ledit second élément gonflable (10).

11. Dispositif selon l'une quelconque des revendications précédentes, dans lequel ledit cathéter (C) comprend en outre une lumière-guide (12') destinée à loger un fil-guide (5) dudit dispositif (1).

12. Dispositif selon la revendication 10, dans lequel ladite lumière-guide (12') coïncide au moins partiellement avec ladite lumière d'auto-perfusion (12).
